# EUROPEAN PATENT APPLICATION

(11) **EP 4 625 380 A1**
(43) Date of publication of application: **01.10.2025**
(21) Application number: 24167864.8
(22) Date of filing: 29.03.2024
(51) Int. Cl.: G08B 21/02, A61B 5/11, A61B 5/00, G08B 21/04, G08B 21/06

(54) **INFANT MONITORING**

(71) Applicant: Koninklijke Philips N.V., 5656 AG Eindhoven (NL)
(72) Inventor: GOSENSHUIS, Daan Hendrik, Eindhoven (NL); BERNTSEN, Luc, Eindhoven (NL); SINGH, Jetendra Kumar, 5656AG Eindhoven (NL)
(74) Representative: Philips Intellectual Property & Standards

(57) **Abstract**

Proposed concepts pertain to determining a sleep status of an infant in a monitored area. In particular, embodiments aim to provide a method for determining the sleep status of a monitored infant by monitoring the infant's level of activity over both a first time period and a second, longer, time period.

## Description

### FIELD OF THE INVENTION

This invention relates to the field of infant monitoring, and in particular to infant video monitoring.

### BACKGROUND OF THE INVENTION

Infant video monitoring systems are often used by parents and carers to monitor an infant whilst they sleep, allowing the parent/carer to check in on the sleeping infant remotely. Infant monitoring systems are often provided with one or more sensors to take visual and/or audio data of a monitored area which may then be relayed to the parent via a separate device or via an app on the parent's smart phone or other personal smart device.

Modern infant monitoring systems may be provided with additional functionalities that track and log the sleep periods of an infant. They may provide additional reassurance or insights to parents on the quantity and quality of sleep their infant is getting.

However, it is difficult to produce accurate results when monitoring the sleep state of an infant using video analysis.

### SUMMARY OF THE INVENTION

The invention is defined by the claims.

According to an aspect of the invention, there is provided a method for determining a sleep status of an infant in a monitored area, wherein the method comprises: processing first video data of the monitored area with a first motion detection algorithm to determine an activity level of the infant in a first time period; processing second video data of the monitored area with a second motion detection algorithm to determine an activity level of the infant in a second time period, wherein the duration of the second time period is greater than the duration of the first time period; and determining, based on the determined activity levels of the infant in the first and second time periods, a sleep status of the infant.

Proposed concepts thus aim to provide schemes, solutions, concepts, designs, methods, and systems pertaining to determining a sleep status of an infant in a monitored area. In particular, embodiments aim to provide a method for determining the sleep status of a monitored infant by monitoring the infant's level of activity over both a first time period and a second, longer, time period.

In other words, it is proposed to use video-based motion detection to determine two measures of the level of activity of a monitored infant which are then used to automatically assess the sleep status of an infant. Within a given period of sleep an infant may pass through various stages of sleep which correspond to different processes happening within the body. The amount an infant is moving (i.e. the infant's activity level) is a key indicator of which stage of sleep the infant is in. Deep sleep is characterized by the infant performing few to no large-scale movements, whereas when awake or in light sleep the infant may move more frequently.

The proposed method may be advantageous when determining the sleep status of a monitored infant in real time. By basing the determination of the infant's sleep status on both the most recent measured movements of the infant, and the infant's activity over a longer time scale, the proposed method has the ability to accurately distinguish between different sleep stages (which may only be possible when motion is analyzed over a longer period of time) whilst also being responsive to recent changes to the infant's sleep status.

Conventional sleep monitoring systems typically only share analytic information on an infant's sleep once the current period of sleep has ended. The infant may be monitored during a sleep session and once the sleep session has been closed, data on the infant's sleep may be processed in a cloud environment and an overview of the infant's sleep status provided to the parent later. In contrast, the presently proposed solution may allow for real-time determination of an infant's sleep status such that a parent may make real-time decisions based on this information. For example, a parent may decide to wait before feeding a sleeping infant if the infant is still in a deep sleep, or the parent may decide not to enter the room in which the infant is sleeping if the infant is still in a light sleep phase.

Using video data for sleep analysis has the advantage that it does not require the infant to wear a wearable tracking device which may be uncomfortable and interfere with the infant's sleep. Further, the proposed method for determining the sleep status does not require the use of an advanced processor and does not require processing in the cloud, being capable of being run on a single core processor. Therefore, the proposed invention may be advantageous in that it requires low computational power and can be run offline.

Real-time determinations of infant sleep status may be unreliable if they are only based on the most recent infant monitoring data. Within a given sleep stage (e.g., light sleep) there may be significant variation in the activity level of the infant. This may lead to false determinations that the sleep status of the infant has changed if only the most recent video data is analyzed. Using only long-term infant monitoring, real-time sleep stage analysis cannot be achieved as the sleep status determination lags behind recent changes to the infant's sleep behavior. It was realized that by using two different time "buffers" to measure the movement activity of the monitored infant, the resulting sleep status may be both responsive to real-time changes in the sleep status of the infant and reliable. The proposed method therefore leverages both the measured activity level of the infant over a first, shorter, time period and the measured infant activity level over a second, longer, time period to determine the sleep status of an infant. The proposed method is therefore capable of producing an accurate, real-time determination of what stage of sleep the infant is in.

Ultimately, an improved method for determining the sleep status of an infant in a monitored area may be supported by the proposed concept(s).

In some embodiments, the sleep status of the infant comprises at least one of: active; awake; light sleep; and deep sleep. The proposed method may thus permit the determination of any of four different possible sleep status values for the monitored infant.

In some embodiments, determining, based on the determined activity levels of the infant in the first and second time periods, a sleep status of the infant comprises: comparing the activity level of the infant in the first time period to a first threshold; and comparing the activity level of an infant in the second time period to a second threshold. Thus, the activity level of the infant may be a quantitative value that can be compared to a predetermined threshold value for the two time periods analyzed to determine what state of sleep the infant is in.

In some embodiments, the method may further comprise: determining the sleep status has a first, awake, value based on comparing the activity level of the infant in the first time period to the first threshold; determining the sleep status has a second, light sleep, value based on comparing the activity level of the infant in the first time period to the first threshold and comparing the activity level of the infant in the second time period to the second threshold; and determining the sleep status has a third, deep sleep, value, based on comparing the activity level of the infant in the first time period to the first threshold and comparing the activity level of the infant in the second time period to the second threshold. This sleep status determination logic allows for the infant to be determined to be awake based only on the infant's movement in the shorter first time period, allowing for real-time monitoring of whether the infant is asleep or not. The activity level of the infant in the second time period can then be used to provide more detailed feedback as to whether the infant is in a light or deep sleep.

In some embodiments, the first time period may be in the range 30 seconds to 3 minutes; and the second time period may be in the range 3 minutes to 10 minutes. Setting the first time period within this range allows for the method to be responsive within a short time frame to changes in the sleep status, whilst setting the longer time period to be in the range 3 to 10 minutes provides a sufficiently long period of monitoring for an accurate distinction between similar sleep status values.

In some embodiments, the activity level of the infant in the first time period may describe the proportion of the first time period in which the infant is moving, and the activity level of the infant in the second time period may describe the proportion of the second time period in which the infant is moving. The proportion of a given time period in which an infant is moving has been shown to be a useful measure of the infant activity level and therefore a useful value for determining the sleep status of an infant.

In some embodiments, video data may comprise a plurality of time sequenced frames and processing video data with a motion detection algorithm may comprise: comparing the pixel values of a first frame of the video data to pixel values of a second frame of the video data to determine a pixel shift signal describing the average change in pixel values between the first and second frames; and determining an activity level of the infant based on the pixel shift signal. Pixel shift analysis has been shown to be a useful method for detecting movement in video data and in particular to determining a measure of the activity level of an infant from video data of the infant.

In some embodiments, comparing pixel values of a first frame of the video data to pixel values of a second frame of the video data may comprise: segmenting the first frame of the video data into a plurality of first blocks; segmenting the second frame of the first video data into a plurality of second blocks corresponding to the first blocks; and determining an average pixel value for each of the plurality of first blocks and each of the plurality of second blocks; and comparing the average pixel value of each of the plurality of first blocks to the average pixel value of each of the plurality of second blocks. Segmenting the frames of the video data into blocks before performing pixel shift analysis may reduce the processing power needed to carry out motion detection on the first and second video data.

In some embodiments, determining an activity level of the infant based on the pixel shift signal comprises: comparing the pixel shift signal to a motion detection threshold value. This step may enable noise in the video data to be filtered out and only large motions of the infant registered as motion detection events. This may provide a more reliable measure of the infant's activity.

In some embodiments, the method may further comprise: processing third video data of the monitored area with a breathing detection model to determine an inter-breath-interval value describing the periodicity of the infant's breathing; and determining the sleep status of the infant, based on the inter-breath-interval value, the activity level of the infant in the first time period, and the activity level of the infant in the second time period. In this way, breathing detection may be used alongside motion detection, resulting in a more accurate determination of the infant's sleep status.

According to another aspect of the present invention, there is provided a computer program comprising code means for implementing any of the methods outlined above when said program is run on a processing system.

According to another aspect of the invention, there is provided a processor arrangement comprising the above computer program and configured to execute the computer program.

According to another aspect of the present invention there is provided a system for determining a sleep status of an infant in a monitored area, the system comprising; an image processing arrangement configured to run the above computer program.

In some embodiments, there may be provided an infant monitoring system comprising the system for determining a sleep status of an infant in a monitored area; and an image sensor configured to obtain said first and second video data of the monitored area. Thus, the processing arrangement and the image sensor may be integrated into a single system.

In some embodiments the infant monitoring system may further comprise an output user interface configured to output the determined sleep status to a user.

Embodiments may be employed in combination with conventional/existing infant monitoring method and systems. In this way, embodiments may integrate into legacy systems and improve and/or extend their functionality and capabilities. An improved infant monitoring system may therefore be provided by proposed embodiments.

Thus, there may be proposed concepts for determining the sleep status of an infant in a monitored area, and this may be done based on determining an activity level of the infant in both a first, shorter time period and a second, longer time period. These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 depicts a simplified flow diagram of a method for determining a sleep status of an infant in a monitored area according to a proposed embodiment;
Fig. 2 depicts a simplified flow diagram of a motion detection algorithm according to a proposed embodiment;
Fig. 3 depicts a simplified flow diagram of a sleep status determination logic employed by a proposed embodiment;
Fig. 4 depicts a simplified flow diagram of a method for determining a sleep status of an infant in a monitored area according to another proposed embodiment;
Fig. 5 is a simplified block diagram of an infant monitoring system for determining the sleep status of an infant in monitored area according to a proposed embodiment; and
Fig. 6 illustrates a simplified block diagram of an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to determining the sleep status of an infant in a monitored area (for example, determining whether an infant in a cot/crib being monitored by a video infant monitor is awake or asleep). According to proposed concepts, several possible solutions may be implemented separately, two or more of these possible solutions may be implemented in one combination or another.

Embodiments of the invention aim to provide a concept for automatically determining the sleep status of an infant from video data. This is achieved by processing the video data with a motion detection algorithm to determine the level of infant activity over both a first time period and a second, longer, time period and then using these determined measures of infant activity to determine the sleep status of the infant. Such a proposed method may be advantageous in that it allows for reliable real-time sleep stage analysis.

By way of summary, an exemplary embodiment comprises the following modules:
A. Obtaining Video Data: an image sensor is configured to capture video data of an infant in a monitored area which may correspond to the bed or crib a sleeping infant is lying on. The image sensor used to obtain the video data may be in the same or a separate device to the device housing image processing capabilities used to carry out the proposed method for determining the sleep status of the infant.
B. Movement Detection: the obtained video data is processed with a motion detection algorithm to track the infant's movement. This allows for the infant's activity level over a given time period to be determined. Specifically, in the proposed invention a first infant activity level is determined describing how much the infant moves in a first time period, and a second infant activity level is determined which describes the how much the infant moves in a second time period. The second time period is set to be longer than the first time period.
C. Sleep Status Logic: based on the determined activity levels of the infant over the two different time periods, sleep status logic is employed to determine the sleep status of the infant. This may include a determination of whether the infant is asleep or awake, and if asleep what stage of sleep they are in (e.g., deep sleep, light sleep, or sleep stages N1, N2, N3, or REM).
D. Sleep Dashboard: The determined sleep status may be displayed to a parent or carer via a smart infant sleep dashboard. The dashboard may inform the user of the current sleep status of the infant or display data indicating historic sleep periods of the infant and the quality of the sleep of the infant in these sleep periods. Further insights into the infant's sleep behavior and patterns may also be provided.

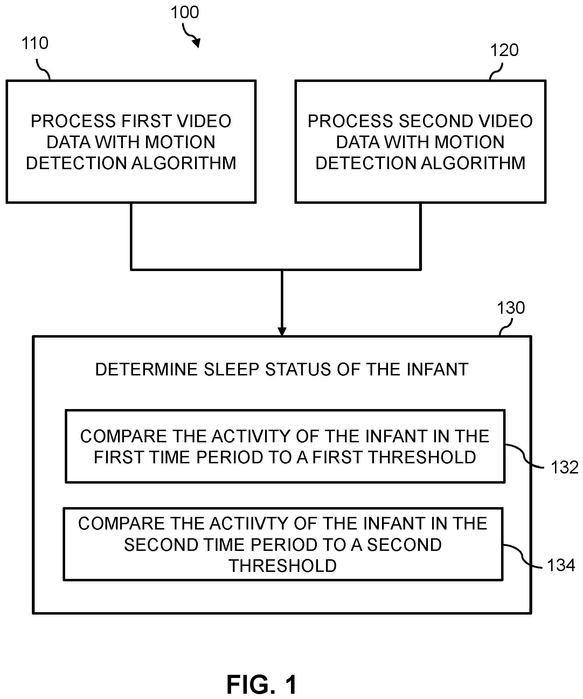

Referring now to Fig. 1, there is depicted a simplified flow diagram of a method 100 for determining the sleep status of an infant in a monitored area.

The method begins with a step 110 comprising processing first video data of the monitored area with a first motion detection algorithm to determine an activity level of the infant in a first time period. In this exemplary embodiment, the first time period has a duration of 2 minutes.

The first video data comprises a series of time-sequenced frames depicting the infant in the monitored area. The motion detection algorithm is used to detect changes to the position, orientation, or pose of the infant between the frames of the first video data. The detected movement is used to determine the activity level of the infant in the first time period. In this exemplary embodiment, the activity level of the infant in the first time period describes the proportion of the first time period in which the infant is moving and may be represented by a percentage, or a numerical value less than or equal to 1. Thus, the motion detection algorithm may monitor infant movement over the first time period and then calculate the percentage of the duration of the first time period for which infant movement is detected.

The step 120 of the method 100 comprises processing second video data of the monitored area with a second motion detection algorithm to determine an activity level of the infant in a second time period, wherein the duration of the second time period is greater than the duration of the first time period. In this exemplary embodiment, the second time period has a duration of 5 minutes and, similar to above, the activity level of the infant in the second time period describes the proportion of the second time period in which the infant is moving. In this example, the method 100 is used to determine the sleep status of the infant in real-time. Hence, the first and second time periods are configured to have a common end point, being the most recent time for which video data of the infant is available, and the first time period fully falls within (i.e. overlaps with) the second time period.

Thus, the method 100 provides an indication of the level of infant movement over both a shorter first timescale and a longer second timescale. The short time scale allows the system to be more responsive to sudden changes in the infant's sleep behavior whilst the longer timescale allows for accurate distinction between different sleep stages.

Once the activity levels of the infant in the first and second time periods have been determined, the method 100 proceeds to a step 130 comprising determining, based on the determined activity levels of the infant in the first and second time periods, a sleep status of the infant. In this embodiment, the sleep status is at least one of awake; active; light sleep; and deep sleep.

In this particular example, the step 130 comprises the sub-steps 132 and 134. Step 132 comprises comparing the activity level of the infant in the first time period to a first threshold and step 134 comprises comparing the activity level of the infant in the second time period to a second threshold. Thus, in this embodiment the method is equipped with a sleep status determination logic based on predetermined threshold values that enable the determination of the infant's sleep status based on the activity level of the infant in a first, shorter, time period and a second, longer, time period. Different sleep stages are associated with different levels of movement of the body therefore by comparing the activity level of the monitored infant to pre-known threshold values it is possible to distinguish between different sleep stages and therefore determine whether the infant is awake, active, in light sleep, or in deep sleep.

Although in this embodiment, the first time period has a duration of 2 minutes and the second time period has a duration of 5 minutes, in other embodiments other suitable values may be used. For example, the first time period may be in any of the ranges: 30 seconds to 3 minutes, 1 minute to 3 minutes, 1 minute to 2 minutes, or 2 minutes to 3 minutes. The second time period may be in any of the ranges: 3 minutes to 10 minutes, 3 minutes to 8 minutes, 3 minutes to 6 minutes, or 4 minutes to 6 minutes. The specific lengths of the first and second time period may be determined by an optimization process. The length of both time periods must be short enough that the system is reactive to sudden changes to the infant's sleep status whilst remaining long enough to allow accurate distinction between similar sleep status values, for example to determine between light and deep sleep of the infant.

Similarly, other aspects of the method 100 may be implemented differently in different embodiments. In the method 100, the first and second time periods are set to be continuous time periods that end at approximately the current time to allow for accurate real-time determination of the sleep status, however in other embodiments the first and second time periods may be configured differently. For example, the first and second time periods may not overlap. The second, longer time period may precede the first time period which may allow for a better determination of the activity of the infant in the long-term. The activity level of the infant may comprise a different metric of the level of infant movement than the proportion of the associated time period in which the infant is moving. For example, an infant activity level may comprise at least one of: the number of detected large movements of the infant, the average rate of infant movements, or the proportion of detected movements that are determined to constitute large-scale movements. Alternatively, the activity level may be a qualitative value describing the level of activity, for example processing video data with the first or second motion detection algorithm may lead to a determination that the infant has "high activity" or "low activity". Other embodiments of the present invention may not rely on the direct comparison of the determined activity levels to predetermined threshold values to determine the sleep status but may perform more complicated calculations on the determined activity levels.

Although described above as first and second video data, the first and second video data in some embodiments may be the same video data which depicts the monitored area over for a duration that fully encompasses both the first and second time periods. For example, when the method 100 is used to determine the sleep status of an infant in real-time, the first and second time periods may both end at the current time and the first and second motion detection algorithm may be the same and may be configured to process video data corresponding to the second time period which fully overlaps with the first time period. Thus, although discussed as first and second motion detection algorithms, in some embodiments these algorithms may be identical. In others, the first and second motion detection algorithms may be different to take into account differences in the first and second video data.

The first and second video data may contain a plurality of time-sequenced video frames. In some embodiments the plurality of time-sequenced frames may be uniformly spaced in time over the entirety of the first and second time periods respectively, but the present invention is not limited to such. Any video data that allows for the determination of the activity level of the infant over the two time periods may be used. For example, in some embodiments the first and second video data may contain discrete snippets of video data. The first video data may comprise a first portion of consecutive time-sequenced frames corresponding to the start of the time period, and a second portion of consecutive time-sequenced frames corresponding to the end of the first time period. Using only partial video data of the first and second time periods in this way may reduce processing requirements. Any suitable video data may be used for this purpose, for example it is proposed to use video data obtained from an infant monitoring camera positioned with a birds-eye view of the infant's sleeping area. In some embodiments, this video data may be cropped to reduce the effects of noise in the surroundings and reduce processing power.

Although the method 100 results in a determination that the sleep status has one of four possible values (awake, active, light sleep, or dark sleep), in other embodiments the method may be configured to determine different values for the sleep status. For example, the sleep status may be at least one of stage N1, stage N2, stage N3, and stage R.

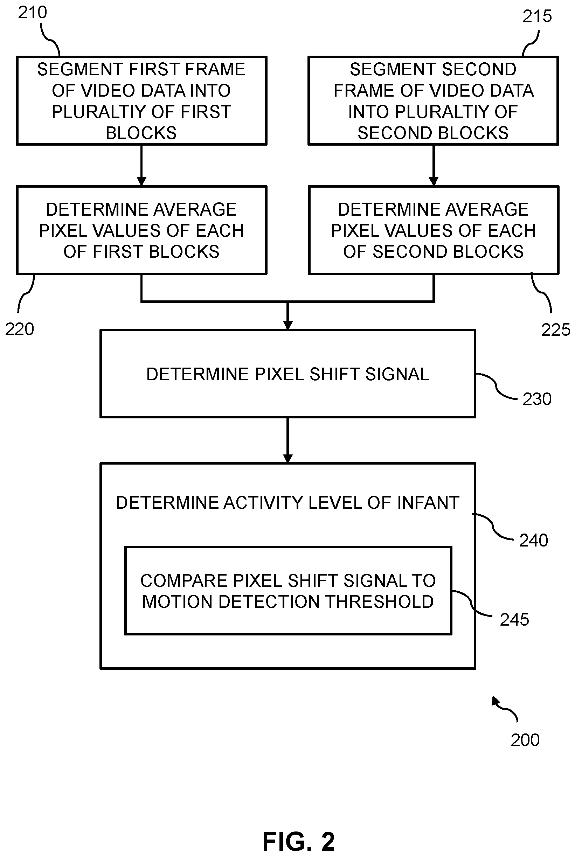

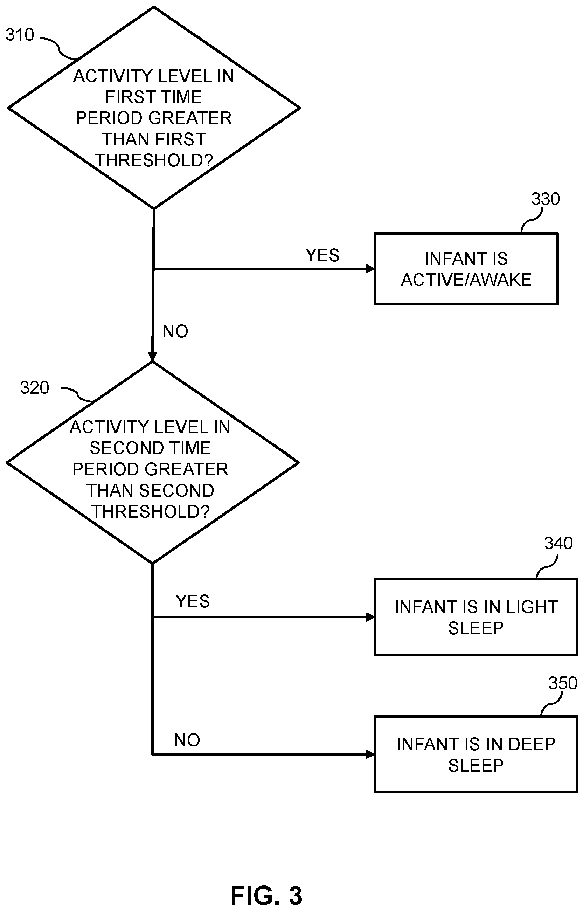

With reference to Figs 2 and 3, further examples are provided of how the different steps of the proposed method may be carried out. Referring now to Fig. 2, there is depicted a simplified flow diagram of a motion detection algorithm 200 according to a proposed embodiment. The motion detection algorithm 200 is used to process video data of an infant in a monitored area to determine an activity level of the infant and may be used within the method 100 to determine the activity levels of the infant in the first and second time periods.

The motion detection algorithm commences with a step 210 of segmenting a first frame of the video data into a plurality of first blocks. Each block may correspond to an 80 pixel by 80 pixel region of the image. Other block sizes may be used depending on the size of the first frame of the video data and the processing power available. Once the first frame has been segmented, the algorithm proceeds to a step 220 in which the average pixel value of each of the first blocks is determined.

In a step 215 a second frame of the video data is segmented into a plurality of second blocks corresponding to the first blocks i.e., the plurality of second blocks may be the same size and shape as the plurality of first blocks. The average pixel value of each of the second blocks is then calculated in a step 225.

The algorithm then proceeds to a step 230 comprising comparing the average pixel value for each of the plurality of first blocks to the average pixel values of each of the plurality of second blocks to determine a pixel shift signal describing the average change in pixel values between the first and second frames of the video data. The pixel shift signal may thus contain information on the difference between the average pixel value of each of the plurality of first blocks and the corresponding block of the plurality of second blocks.

The algorithm then proceeds to a step 240 comprising determining an activity level of the infant based on the pixel shift signal. In this exemplary embodiment, the step 240 further comprises the sub-step 245 comprising comparing the pixel shift signal to a motion detection threshold.

In detail, if the pixel shift signal is greater than the motion detection threshold it may be determined that the infant has performed a large-scale motion between the first and second frames of the video data, and if the pixel shift signal is less than the motion detection threshold it may be determined that the infant has not performed a large-scale motion between the first and second frames of the video data. By using a motion detection threshold in this way, the algorithm filters out noise and small-scale movements not related to infant activity such as those produced by infant breathing. The first and second frames may be consecutive, or non-consecutive frames. The frame rate of the video data may be at least one of: 8 fps, 10 fps, and 12 fps. The algorithm described above may be repeated on a plurality of time-sequenced frames of the video data to determine at various time intervals within the duration of the video data, whether the infant has performed any large scale motions or not. The activity level of the infant may then be calculated from these detected motions.

The segmentation-based pixel-shift algorithm described above may be particularly advantageous in that processing requirements are reduced. However, this is just one possible way in which the motion detection algorithm may be implemented. Other known motion detection algorithms may be used to monitor and track infant movement events over a pre-determined time period. In accordance with the present invention, these may then be used to determine a measure of the activity level of the infant.

Referring now to Fig. 3, there is depicted a simplified flow diagram of a sleep status determination logic employed by a proposed embodiment. This may be employed to determine the sleep status of an infant from the determined activity levels of the infant in a first time period and a second, longer time period.

In a first decision step 310, it is determined whether the activity level of the infant in the first time period is greater than a first threshold. If this activity level is greater than the first threshold than the infant is determined to be active and/or awake. In some applications of the present invention, it may not be possible to distinguish between an infant that is highly active and one that is awake without further determination of whether the infant has their eyes open. Therefore, the sleep status may be determined to have an active/awake value.

In this exemplary embodiment, the infant activity level in the first time period represents the percentage of a 2 minute period in which the infant moves, and the first threshold is set at 50%. The infant is therefore deemed to be awake/active if the first motion detection algorithm detects infant movement in greater than 50 % of the past 2 minute period.

If the activity level of the infant in the first time period is less than the first threshold (i.e. the infant is detected to move for less than 50% of the 2 minute period), the method moves to a second determination step 320. This step comprises asking if the activity level of the infant in the second time period is greater than a second threshold. If the activity level in the second, longer time period is greater than the second threshold, then the infant is determined to be in a light sleep state, and if this activity level is determined to be lower than the second threshold the infant is deemed to be in a deep sleep state. In this example, the infant activity level in the second time period is the percentage of a 5 minute time period for which the infant is detected to move. The second threshold is set at 7.5%, such that if the infant is detected to move in greater than 7.5% of the 5 minute time period and less than 50% of the 2 minute time period they are deemed to be in a light sleep stage, and if the infant is detected to move in less than 7.5 % of the 5 minute time period and less than 50% of the 2 minute time period they are deemed to be in a deep sleep stage.

The sleep status determination logic shown in Fig. 3 is just one example of the logic that may be employed by a method of the present invention, and the present invention is not limited to such. Many other sleep status logics using different measures of the activity level of the infant, different lengths of the first and second time period, and different threshold values may be utilized depending on exact specifications of the video data and motion detection algorithms used.

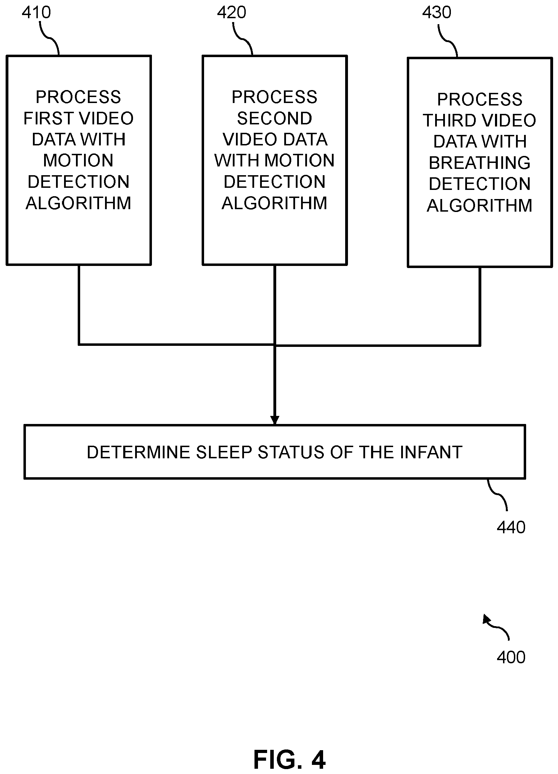

Referring now to Fig. 4, there is depicted a simplified flow diagram of a method 400 for determining a sleep status of an infant in a monitored area according to another proposed embodiment.

The method 400 commences with a step 410 (equivalent to step 110 of the method 100) comprising processing first video data with a first motion detection algorithm to determine an activity level of the infant in a first time period.

The step 220 is equivalent to step 120 of method 100 and comprises processing second video data with a second motion detection algorithm to determine an activity level of the infant in a second time period, wherein the duration of the second time period is longer than the duration of the first time period.

The method 400 further comprises the step 430 comprising processing third video data of the monitored area with a breathing detection model to determine an inter-breath interval value describing the periodicity of the infant's breathing. The third video data may be the same as the first and/or second video data and as such the inter-breath-interval value may describe the average time period between infant breaths over the first and/or second time period. Alternatively, the inter-breath-interval may be calculated over a third time period, different from the first and second time period.

Once the activity levels of the infant in the first and second time period, and the inter-breath-interval value of the infant have been calculated, the method 400 commences to a step 440. This step comprises determining the sleep status of the infant based on the inter-breath interval value, the activity level of the infant in the first time period, and the activity level of the infant in the second time period.

This method, in which inter-breath-interval variability is used alongside activity level determination, may result in a more accurate determination of the infant's sleep status. High or low variation in the inter-breath-interval value may be a good determining factor to distinguish between a deep non-REM sleep or an active REM-like sleep. Breathing detection may be achieved through a pixel-shift motion detection algorithm specifically configured to detect small periodic motions. The time period between detected breaths may then be measured and the variability in this quantity analyzed to determine the inter-breath interval value. The sleep status determination may then depend on all three of the inter-breath interval value, the activity level of the infant in the first time period, and the infant activity level in the second time period, to improve the accuracy of this determination.

Similarly to the method 400, in alternative embodiments the method 100 may be extended to include other video processing techniques to improve the accuracy of the sleep status determination. For example, a feature detection algorithm may be used to process video data of the infant to identify certain facial features. This may result in the determination of information describing whether the infant's eyes are open or closed which may allow for determining whether the infant is active but asleep, or whether the infant is awake when large amounts of infant movement are detected.

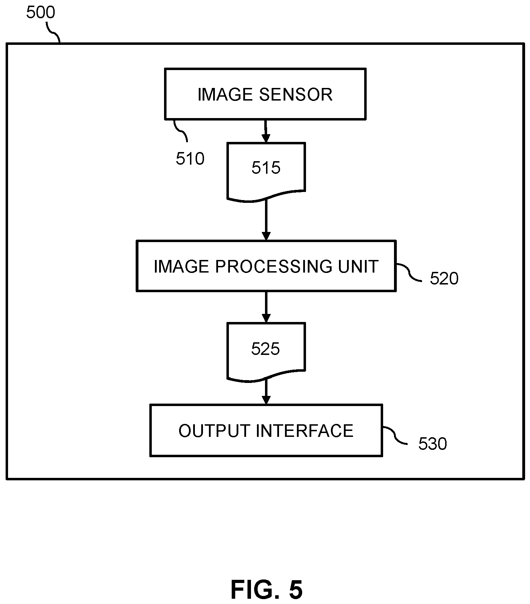

Referring now to Fig. 5, there is depicted a simplified block diagram of an infant monitoring system 500 for determining the sleep status of an infant in monitored area according to a proposed embodiment. The infant monitoring system comprises an image sensor 510, an image processing unit 520, and an output interface 530. The image processing unit 520 may also be referred to as a processor arrangement.

The system 500 is configured to determine the sleep status of an infant that is being monitored by the system. It achieves this by processing in the image processing unit 520 video data from the image sensor 510. This may be carried out using any above proposed methods of the invention.

In detail, the image sensor 510 obtains video data 515 comprising first and second video data of an infant present in a monitored area. The first and second video data may in some embodiments be the same video data. The first and second video data may comprise only portions of the video data obtained by the image sensor, for example the first and second video data may be cropped compared to the video data obtained by the image sensor 510 and/or the first and second video data may comprise only a portion of the frames of the video data obtained by the image sensor 510. In other sleep monitoring systems, the first and second video data may be obtained from different image sensors.

The image processing unit 530 is configured to run computer code comprising code means for implementing the method for determining the sleep status of an infant according to any proposed embodiment. Thus, on receiving the video data 515 the image processing unit 530 processes the first video data with a first motion detection algorithm to determine an activity level of the infant in a first time period. The image processing unit also processes the second video data of the monitored area with a second motion detection algorithm to determine an activity level of the infant in a second time period, the duration of the second time period being greater than the duration of the first time period. The image processing unit is the further configured to determine, based on the determined activity, a sleep status of the infant.

The image processing unit 520 is configured to output a signal 525 comprising the determined sleep status to the output interface 530 which may then output this status to the user (e.g. as an electronic output signal and/or a visible/audible output). In some embodiments, a signal containing information describing the sleep status may be output to a device external to the infant monitoring system. This may allow for the sleep status to be used to adjust aspects of the infant's environment to improve the infant's sleep. This may include adjusting the lighting near the infant's crib or, for cribs equipped with automated rocking, the level of movement of the infant's crib.

The output interface 540 may be further configured to output more detailed information describing an infant's sleep to aid parents in assessing and monitoring the sleep of their infant. For example, the output interface may be configured to display a sleep dashboard to the user. The dashboard may include a live video of an infant lying in the crib. The video streaming can be turned on or off depending on the requirements of the parents and the resources available in the device. Several features related to different aspects of infant sleep may be tracked and communicated by the dashboard in real time such as: whether infant is present in crib; infant awake or asleep status; infant sleep stage status; infant breathing status (if yes, an indication of whether breathing is within normal range may also be provided); and audible and/or visible alert(s) if the infant is detected to be absent and/or when there is an anomaly in breathing rate.

The dashboard may thus provide high-level analytics/insights regarding the infant's sleep habits (based on the sleep history for example). For example, a bar chart of total sleep duration in past seven days and the quality of this sleep may be displayed to a user. Such data can support the parents in determining if the sleep cycle of the infant is being affected when there is an anomaly in the chart. The bar chart may also extend to providing the sleep duration in past two weeks or past month.

Although in the system 500, the image sensor 510, the image processing unit 520, and the output interface 530 are present in the same device, it will be understood that these components may be distributed among various devices. For example, there may be provided an infant monitor comprising only an image sensor. The video data obtained by the image sensor of the infant monitor may then be relayed to a personal smart device of the parent/carer. The processing and interface capacities of the personal smart device may then fulfil the functionalities of the image processing unit 520 and the output interface 530.

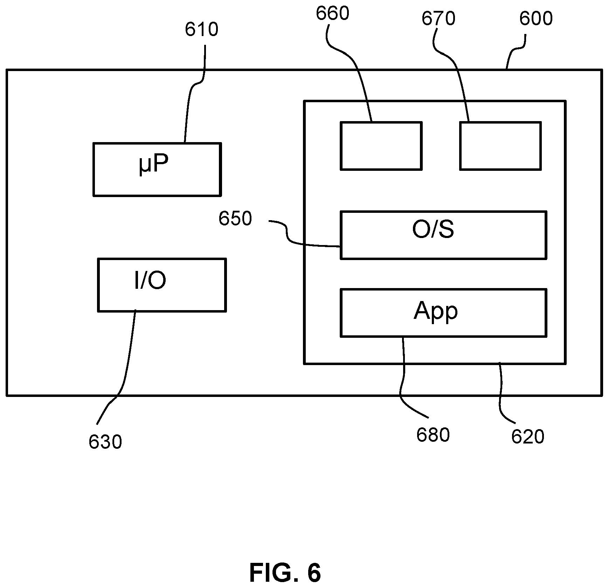

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that area communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor-based microprocessor (in the form of a microchip or a microprocessor).

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs 1 and 4, the logic of Fig. 2, the algorithm of Fig. 3, and the system of Fig. 5, may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrate d in the flow diagrams - may be performed by the same or different computing devices. According to embodiments, a computer-readable storage medium stores a computer program comprising computer program code configured to cause one or more physical computing device to carry out a method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figs 5 and 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementation of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending on the functionality involved. It will also be noted that each block of the block diagrams and/or flow diagrams, and combinations of blocks in the block diagrams and/or flow diagrams, can be implemented by special purpose hardware-based systems that perform the specified instructions the architecture, functionality, and operation of possible implementations of systems, methods, and computer program products according to various embodiments of the present invention. In this regard, each block in the flow diagrams or block diagrams may represent a module segment, or portion of instructions, which comprises one or more executable instructions for implementing the specified logical function(s).

## Claims

1. A method for determining a sleep status of an infant in a monitored area, wherein the method comprises:
processing first video data of the monitored target area with a first motion detection algorithm to determine an activity level of the infant in a first time period;
processing second video data of the monitored target area with a second motion detection algorithm to determine an activity level of the infant in a second time period, wherein the duration of the second time period is greater than the duration of the first time period; and
determining, based on the determined activity levels of the infant in the first and second time periods, a sleep status of the infant.

2. The method of claim 1, wherein the sleep status of the infant comprises at least one of: awake; active; light sleep; and deep sleep.

3. The method of any preceding claim, wherein determining, based on the determined activity levels of the infant in the first and second time periods, a sleep status of the infant comprises:
comparing the activity level of the infant in the first time period to a first threshold; and
comparing the activity level of the infant in the second time period to a second threshold.

4. The method of claim 3, further comprising one of:
determining the sleep status has a first, awake, value based on comparing the activity level of the infant in the first time period to the first threshold;
determining the sleep status has a second, light sleep, value based on comparing the activity level of the infant in the first time period to the first threshold and comparing the activity level of the infant in the second time period to a second threshold; and
determining the sleep status has a third, deep sleep, value based on comparing the activity level of the infant in the first time period to the first threshold and comparing the activity of the infant in the second time period to a second threshold.

5. The method of any preceding claim, wherein:
the first time period is in the range 30 seconds to 3 minutes; and
the second time period is in the range 3 minutes to 10 minutes.

6. The method of any preceding claim, wherein:
the activity level of the infant in the first time period describes the proportion of the first time period in which the infant is moving, and
the activity level of the infant in the second time period describes the proportion of the second time period in which the infant is moving.

7. The method of any preceding claim, wherein video data comprises a plurality of time sequenced frames and processing video data with a motion detection algorithm comprises:
comparing the pixel values of a first frame of the video data to pixel values of a second frame of the video data to determine a pixel shift signal describing the average change in pixel values between the first and second frames; and
determining an activity level of the infant based on the pixel shift signal.

8. The method of claim 7, wherein comparing pixel values of a first frame of the video data to pixel values of a second frame of the video data comprises:
segmenting the first frame of the video data into a plurality of first blocks;
segmenting the second frame of the first video data into a plurality of second blocks corresponding to the first blocks; and
determining an average pixel value for each of the plurality of first block and each of the plurality of second blocks; and
comparing the average pixel value of each of the plurality of first blocks to the average pixel values of each of the plurality of second blocks.

9. The method of any of claims 7 and 8, wherein determining an activity level of the infant based on the pixel shift signal comprises:
comparing the pixel shift signal to a motion detection threshold value.

10. The method of any preceding claim, wherein the method further comprises:
processing third video data of the monitored area with a breathing detection model to determine an inter-breath-interval value describing the periodicity of the infant's breathing; and
determining the sleep status of the infant, based on the inter-breath-interval value, the activity level of the infant in the first time period, and the activity level of the infant in the second time period.

11. A computer program comprising code means for implementing the method of any preceding claim when said program is run on a processing system.

12. A processor arrangement comprising the computer program of claim 11 and configured to execute the computer program.

13. A system for determining a sleep status of an infant in a monitored area, the system comprising:
an image processing arrangement configured to run the computer program of claim 11.

14. An infant monitoring system comprising:
the system of claim 13; and
an image sensor configured to obtain said first and second video data of the monitored area.

15. The infant monitoring system of claim 14, further comprising an output user interface configured to output the determined sleep status to a user.
